# EUROPÄISCHE PATENTSCHRIFT

(11) **EP 1 385 972 B1**
(45) Veröffentlichungstag und Bekanntmachung des Hinweises auf die Patenterteilung: **08.10.2008**
(21) Anmeldenummer: 02730207.4
(22) Anmeldetag: 25.04.2002
(51) Int. Cl.: C12P 13/02, C12M 1/02, C07C 231/06, B01J 14/00

(54) **VERFAHREN ZUR HERSTELLUNG EINER WÄSSRIGEN ACRYLAMIDLÖSUNG MIT EINEM BIOKATALYSATOR**
METHOD FOR PRODUCING AN AQUEOUS ACRYLAMIDE SOLUTION WITH A BIOCATALYST
PROCEDE DE PRODUCTION D'UNE SOLUTION D'ACRYLAMIDE AQUEUSE AVEC UN BIOCATALYSEUR

(30) Priorität: 26.04.2001 DE 10120550
(43) Veröffentlichungstag der Anmeldung: 04.02.2004
(73) Patentinhaber: Ashland Licensing and Intellectual Property LLC, Dublin, OH 43017 (US)
(72) Erfinder: PETERSEN, Olaf, 60667 Meerbusch (DE); THEIS, Burkhard, 47447 Moers (DE); COLBERG, Michael, 47877 Willich (DE)
(74) Vertreter: Wolff, Felix
(86) Internationale Anmeldenummer: PCT/EP2002/004567
(87) Internationale Veröffentlichungsnummer: WO 2002/088373

(56) Entgegenhaltungen:
- EP-A- 1 046 706
- DE-A- 3 017 005
- GB-A- 2 054 563
- US-A- 4 181 576
- US-A- 5 604 132
- US-A- 5 811 595
- DATABASE WPI Section Ch, Week 199954 Derwent Publications Ltd., London, GB; Class A41, AN 1999-631952 XP002210901 & RU 2 112 804 C (PERM KIROV WKS), 10. Juni 1998 (1998-06-10)
- STEPHANOPOULOS, G. (EDITOR): "Biotechnology Vol. 3, Bioprocessing" 1993 , VCH VERLAGSGESELLSCHAFT , WEINHEIM XP002210899 Seite 83 Seite 321 -Seite 324
- HWANG, JUN SIK ET AL.: BIOTECHNOLOGY AND BIOENGINEERING, Bd. 34, 1989, Seiten 380-386, XP001040408
- BRUNCH, ALAN WILLIAM: ANTONIE VAN LEEUWENHOEK, Bd. 74, 1998, Seiten 89-97, XP001091362
- WALAS, STANLEY M.: "Chemical Process Equipment - Selection and Design" 1988 , BUTTERWORTHS PUBLISHERS , BOSTON XP002210900 Seite 45

## Beschreibung

Die vorliegende Erfindung betrifft ein Verfahren und eine Vorrichtung zur Herstellung einer wässrigen Acrylamidlösung durch Hydratisierung von Acrylnitril in einer wässrigen Lösung in Gegenwart eines Biokatalysators.

Die Umsetzung von Acrylnitril zu Acrylamid in Gegenwart eines geeigneten Biokatalysators in Wasser ist selt vielen Jahren bekannt und wird beispielsweise in der DE 30 17 005 C2 beschrieben, wobei bei diesem Verfahren der Biokatalysator immobilisiert ist. In der DE 44 80132 C2 und in der EP 0 188 316 B1 werden spezielle Biokatalysatoren zur Umsetzung von Acrylnitril in Acrylamid beschrieben. Die US 5,334,519 lehrt die Hydratisierung von Acrylnitril zu Acrylamid in der Gegenwart von Biokatalysatoren und Kobalt-Ionen. All diese Lehren haben den Nachteil, daß der Biokatalysator bei der Reaktion geschädigt wird, so daß dessen Aktivität vermindert wird bzw. vermehrt ungewünschte Nebenprodukte entstehen.

GB 2 054 563 A betrifft die Synthese von Acrylamid durch einen Mikroorganismen, wobei die kühlung der Reaktionsmischung vorzugsweise mit Hilfe von Eis erfolgt. EP 1 046 706 A1 betrifft ein Verfahren und eine Vorrichtung zur kontinuierlichen biokatalytischen Umsetzung geeigneter Edukte in wässriger lösung.

Aufgabe der vorliegenden Erfindung ist es deshalb, ein Verfahren zur Verfügung zu stellen, bei dem der Biokatalysator während der Reaktion möglichst wenig geschädigt, Nebenprodukte minimiert und die Batch-Zeit optimiert wird.

Die Aufgabe wird erfindungsgemäß durch ein Verfahren zur Herstellung einer wässrigen Acrylamidlösung durch Hydratisierung von Acrylnitril in einer wässrigen Lösung in Gegenwart eines Biokatalysators gelöst, bei dem das Reaktionsgemisch durchmischt wird, die Hydratisierung in einem Reaktor durchgeführt wird, der Reaktor einen Umpumpkreislauf aufweist, in dem ein Teil des Reaktionsgemisches mit einer Pumpe im Kreis gefördert wird und in dem mindestens ein Wärmetauscher angeordnet ist, **dadurch gekennzeichnet, dass** die Zugabe des Acrylnitrils in den Umpumpkreislauf unmittelbar vor dem Wiedereintritt des Reaktionsgemischs in den Reaktor erfolgt und wobei der Reaktionsverlauf durch On-Line-Messung überwacht wird, in dem die Acrylnitril-und/oder Acrylamidkonzentration im Umpumpkreislauf vor der Zugabestelle des Acrylnitrils gemessen wird.

Zum Reaktionsstart werden in dem Reaktor vollentsalztes Wasser und der Biokatalysator vorgelegt und auf eine Temperatur von 15 bis 25 °C, vorzugsweise 16 bis 20 °C, gebracht. Nachdem die Temperatur erreicht ist, wird das Acrylnitril in den Reaktor dosiert und die Umsetzung zu Acrylamid beginnt. Vorzugsweise erfolgt die gesamte Umsetzung isotherm. Die Konzentration der Biomasse angegeben in Trockensubstanz beträgt bei Reaktionsbeginn vorzugsweise 0,03 - 2,5 g/l, besonders bevorzugt 0,05 -1g/l und der pH-Wert vorzugsweise 6,0 - 8,0, besonders bevorzugt 6,5 - 7,5.

In dem Reaktor ist vorzugsweise ein intensiv wirkendes Rührorgan angeordnet, mit dem der Reaktorinhalt homogen durchmischt wird. In einer bevorzugten Ausführungsform weist der Reaktor außenliegende Halbrohrschlangen auf, mit denen das Reaktionsgemisch während der Umsetzung von Acrylnitril zu Acrylamid zusätzlich gekühlt werden kann.

Erfindungsgemäß weist der Reaktor einen Umpumpkreislauf auf, in dem ein Teil des Reaktionsgemisches mit einer Pumpe im Kreis gefördert wird. In diesem Umpumpkreislauf ist mindestens ein Wärmetauscher angeordnet, mit dem die Reaktionswärme abgeführt werden kann. Vorzugsweise ist der Wärmetauscher ein Rohrbündelwärmetauscher, in dem das Reaktionsgemisch vorteilhafterweise nicht umgelenkt wird, um Fouling an den Wärmetauscheroberflächen zu vermelden.

In einer bevorzugten Ausführungsform der vorliegenden Erfindung werden die Pumpe und der/die Wärmetauscher so ausgelegt, daß zum einen Temperaturschwankungen im Reaktor und zum anderen ein zu starker Energieeintrag durch die Pumpe vermieden wird. Vorzugsweise ist die Pumpe eine magnetisch gekuppelte Seitenkanalpumpe.

Die Zugabe des Acrylnitrils in dem Umpumpkreislauf erfolgt unmittelbar vor dem Wiedereintritt des Reaktionsgemisches in den Reaktor. Die Dosierung wird vorzugsweise kontinuierlich vorgenommen. Als Dosierpumpe für das Acrylnitril hat sich eine frequenzgesteuerte Kolben-Membranpumpe als besonders vorteilhaft erwiesen,

Nach Beendigung der Acrylnitrildosierung ist eine Nachreaktion von vorzugsweise 4 bis 20 Minuten, besonders bevorzugt 5 bis 10 Minuten, erforderlich, um das Acrylnitril möglichst vollständig umzusetzen. Während dieser Nachreaktionszeit ist es vorteilhaft. wenn die Kühlung mit dem Bypass sukzessive reduziert wird.

Zur Optimierung der Reaktionsführung wird der Reaktionsverlauf in dem Reaktor durch eine On-Line-Messung überwacht. Durch diese Messung ist es möglich, die Reaktionsführung sehr schnell an mögliche Veränderungen anzupassen. Die On-Line-Messung in dem Umpumpkreislauf erfolgt vor der Zugabestelle des Acrylnitrils und es werden die Acrylnitril- und/oder die Acrylamidkonzentration fortlaufend überwacht.

Als vorteilhaft hat sich eine On-Line-Messung mit einem Fourier-Transformations-Infrarot-Gerät (FT-IR Gerät) erwiesen.

Die Meßergebnisse der On-Line-Messung können zur Steuerung der Umsetzung herangezogen werden. Vorteilhafterweise werden die Acrylnitrilzugabemenge, die Größe des Umpumpstroms, die Bypass-Menge und die Nachreaktionszeit gesteuert.

Das erfindungsgemäße Verfahren kann mit jedem Biokatalysator durchgeführt werden, der die Umsetzung von Acrylnitril zu Acrylamid katalysiert. Vorzugsweise ist der Biokatalysator jedoch ein Rhodococcus rhodochrous, der unter der Hinterlegungsbezeichnung 14230 bei der DSMZ, Deutsche Sammlung von Mikroorganismen und Zellkulturen GmbH, Mascheroder Weg 1b, D-38124 Braunschweig, Deutschland, hinterlegt ist

Das erfindungsgemäße Verfahren hat den Vorteil, daß der Biokatalysator während der Umsetzung des Acrylnitrils zu Acrylamid möglichst wenig geschädigt und daher die Menge des einzusetzenden Biokatalysators minimiert wird, daß weniger Nebenprodukte anfallen, daß die Umsetzung des Acrylnitrils zumindest nahezu vollständig erfolgt und daß eine bis zu 50 Gew.-%ige Acrylamidlösung erzielbar ist. Das erfindungsgemäße Verfahren ist einfach und kostengünstig durchzuführen. Die Reaktionszeiten können mit dem erfindungsgemäßen Verfahren drastisch reduziert werden. Der Biokatalysator wird optimal ausgenutzt.

Das erfindungsgemäße Verfahren wird vorzugsweise in einer Vorrichtung zur Herstellung einer wässrigen Acrylamidlösung durch Hydratisierung von Acrylnitril in einer wässrigen Lösung in Gegenwart eines Biokatalysators mit einem Reaktor, einem Umpumpkreislauf, in dem ein Teil des Reaktorgemisches von einer Pumpe im Kreis gefördert wird und mindestens einem Wärmetauscher, der in dem Umpumpkreislauf angeordnet ist, durchgeführt. Diese Vorrichtung ist deshalb ein weiterer Gegenstand der vorliegenden Erfindung.

In dem Reaktor ist vorzugsweise ein intensiv wirkendes Rührorgan angeordnet, mit dem der Reaktorinhalt homogen durchmischt wird. In einer bevorzugten Ausführungsform weist der Reaktor außenliegende Halbrohrschlangen auf, mit denen das Reaktionsgemisch während der Umsetzung von Acrylnitril zu Acrylamid zusätzlich gekühlt werden kann.

Erfingdungsgemäß weist der Reaktor einen Umpumpkreislauf auf, in dem ein Teil des Reaktionsgemisches mit einer Pumpe im Kreis gefördert wird. In diesem Umpumpkreislauf ist mindestens ein Wärmetauscher angeordnet, mit dem die Reaktionswärme abgeführt werden kann. Vorzugsweise ist der Wärmetauscher ein Rohrbündelwärmetauscher, in dem das Reaktionsgemisch vorteilhafterweise nicht umgelenkt wird, um Fouling an den Wärmetauscheroberflächen zu vermeiden.

In einer bevorzugten Ausführungsform der vorliegenden Erfindung werden die Pumpe und der/die Wärmetauscher so ausgelegt, daß zum einen Temperaturschwankungen im Reaktor und zum anderen ein zu starker Energieeintrag durch die Pumpe vermieden wird. Vorzugsweise ist die Pumpe eine Seitenkanalpumpe.

Die Zugabe des Acrylnitrils in dem Umpumpkreislauf erfolgt unmittelbar vor dem Wiedereintritt des Reaktionsgemisches in den Reaktor. Die Dosierung wird vorzugsweise kontinuierlich vorgenommen. Als Dosierpumpe für das Acrylnitril hat sich eine frequenzgesteuerte Kolben-Membranpumpe als besonders vorteilhaft erwiesen.

Nach Beendigung der Acrylnitrildosierung ist eine Nachreaktion von vorzugsweise 4 bis 20 Minuten, besonders bevorzugt 5 bis 10 Minuten, erforderlich, um das Acrylnitril möglichst vollständig umzusetzen. Während dieser Nachreaktionszeit ist es vorteilhaft, wenn die Kühlung mit mindestens einem Bypass sukzessive reduziert wird.

Zur Optimierung der Reaktionsführung wird der Reaktionsverlauf in dem Reaktor vorteilhafterweise durch eine On-Line-Messung überwacht. Durch diese Messung ist es möglich, die Reaktionsführung sehr schnell an mögliche Veränderungen anzupassen. Die On-Line-Messung in dem Umpumpkreislauf erfolgt vor der Zugabestelle des Acrylnitrils und es werden die Acrylnitril- und/oder die Acrylamidkonzentration fortlaufend überwacht.

Als vortellhaft hat sich eine On-Line-Messung mit einem Fourier-Transformations-Infrarot-Gerät (FT-IR Gerät) erwiesen.

Die Meßergebnisse der On-Line-Messung können zur Steuerung der Umsetzung herangezogen werden. Vorteilhafterweise werden die Acrylnitrilzugabemenge, die Größe des Umpumpstroms, die Bypass-Menge und die Nachreaktionszeit gesteuert.

Die erfindungsgemäße Vorrichtung hat den Vorteil, daß der Biokatalysator während der Umsetzung des Acrylnitrils zu Acrylamid möglichst wenig geschädigt und daher die Menge des einzusetzenden Biokatalysators minimiert wird, daß weniger Nebenprodukte anfallen, daß die Umsetzung des Acrylnitrils zumindest nahezu vollständig erfolgt und daß eine bis zu 50 Gew.-%ige Acrylamidlösung erzielbar ist. Die erfindungsgemäße Vorrichtung ist einfach und kostengünstig durchzuführen. Die Reaktionszeiten können mit dem erfindungsgemäßen Verfahren drastisch reduziert werden. Der Biokatalysator wird optimal ausgenutzt.

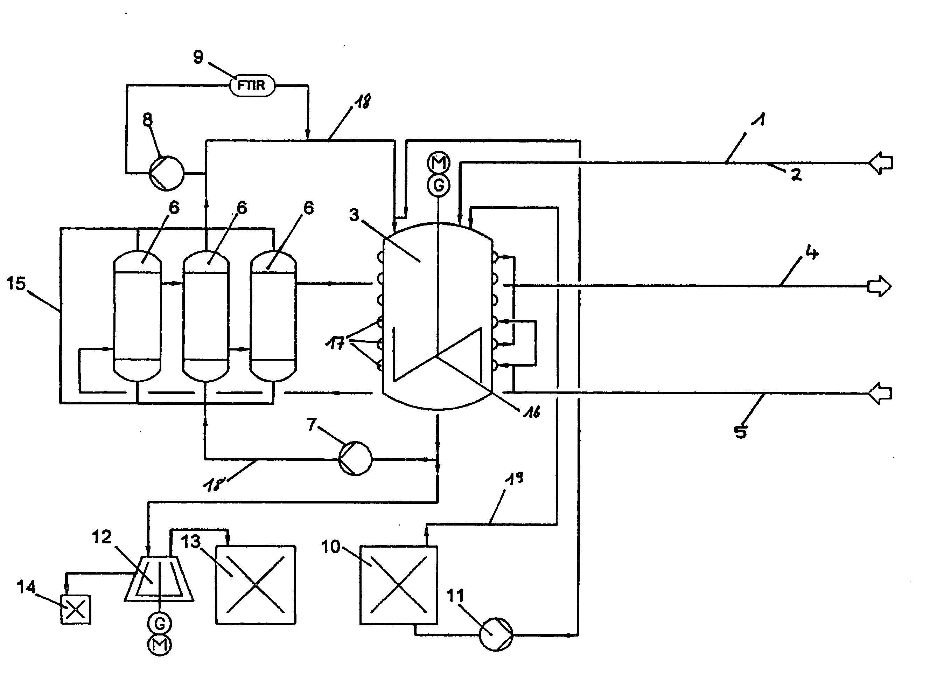

Im folgenden wird die Erfindung anhand Figur 1 erläutert. Diese Erläuterungen sind lediglich beispielhaft und schränken den allgemeinen Erfindungsgedanken nicht ein.

Figur 1 zeigt ein Verfahrensschema des erfindungsgemäßen Verfahrens bzw. Teile der erfindungsgemäßen Vorrichtung. Vor dem Beginn der eigentlichen Umsetzung des Acrylnitrils zu Acrylamid wird in den Reaktor 3 vollentsalztes Wasser 1 und eine Suspension 2, die den Biokatalysator enthält, vorgelegt. Der Inhalt des Reaktors 3 wird mit einem motorgetriebenen Rührer 16 homogen durchmischt. An der Außenseite des Reaktors 3 sind Kühlschlangen 17, die mit dem Kaltwasservorlauf 5 und dem Kaltwasserrücklauf 4 verbunden sind. Der Fachmann erkennt, daß mit diesen Kühlschlangen auch der Reaktorinhalt vor dem Beginn der eigentlichen Reaktion auf eine bestimmte Temperatur vorgewärmt werden kann.

Des weiteren weist der Reaktor 3 einen Umpumpkreislauf 18 auf, durch den ein Teil des Reaktorinhalts mittels der magnetisch gekuppelten Seitenkanalpumpe 7 im Kreis gefördert wird. In dem Umpumpkreislauf 18 sind drei parallel geschaltete Rohrbündelwärmetauscher 6 angeordnet, mit denen der Reaktorinhalt erwärmt bzw. gekühlt werden kann. Die Wärmetauscher 6 sind ebenfalls mit der Kaltwasservorlauf bzw. - rücklauf in Reihe verbunden. Weiterhin weist der Umpumpkreislauf den Bypass 15 auf, mit dem die Wärmetauscher 6 umfahren werden können. Die entsprechenden Ventile sind nicht dargestellt. In dem Umpumpkreislauf ist außerdem das Fourier-Transformations-Infrarot-Gerät (FT-IR Gerät) 9 zur Online-Messung der Acrylnitril-und der Acrylamidkonzentration in dem Umlaufstrom 18 und damit in dem Reaktor 3 gemessen. Der Probenstrom wird dem Umpumpkreislauf 18 entnommen und mit der Kolben-Membranpumpe 8 in das FT-IR Gerät 9 gefördert und dort analysiert. Die Analysedaten werden zur Steuerung des Verfahrens herangezogen. Kurz bevor der Umpumpkreislauf 18 wieder in den Reaktor 3 eintritt, wird ihm das umzusetzende Acrylnitril mit der Membrandosierpumpe 11 aus der Acrylnitrilvorlage 10 zudosiert. Die Acrylnitrilvorlage 10 und der Reaktor 3 sind über eine Pendelleitung 19 gasseitig miteinander verbunden. Die Leitung 19 wird vor dem Dosierungsbeginn des Acrylnitrils geöffnet und nach Beendigung der Dosierung wieder geschlossen. Nach Abschluß der Reaktion wird das wässrige Acrylamid mit einer Ringspaltzentrifuge 12 von der Biomasse getrennt und das wässrige Acrylamid in der Vorlage 13 und die Biomasse in der Vorlage 14 aufgefangen.

## Patentansprüche

1. Verfahren zur Herstellung einer wässrigen Acrylamidlösung durch Hydratisierung von Acrylnitril in einer wässrigen Lösung in Gegenwart eines Biokatalysators, wobei das Reaktionsgemisch durchmischt wird, die Hydratisierung in einem Reaktor durchgeführt wird, der einen Umpumpkreislauf aufweist, in dem ein Teil des Reaktionsgemisches mit einer Pumpe im Kreis gefördert wird und in dem mindestens ein Wär metauscher angeordnet ist, **dadurch gekennzeichnet, dass** die Zugabe des Acrylnitrils in dem Umpumpkreislauf unmittelbar vor dem Wiedereintritt des Reaktionsgemischs in den Reaktor erfolgt und wobei der Reaktionsverlauf durch On-Line-Messung überwacht wird, indem die Acrylnitril- und/oder die Acrylamidkonzentration im Umpumpkreislauf vor der Zugabestelle des Acrylnitrils gemessen wird.

2. Verfahren nach Anspruch 1, **dadurch gekennzeichnet, dass** der Reaktor eine Kühlung, vorzugsweise außenliegende Halbrohrschlangen, aufweist.

3. Verfahren nach Anspruch 1 oder 2, **dadurch gekennzeichnet, dass** der Wärmetauscher ein Rohrbündelwärmetauscher ist, in dem das Reaktionsgemisch gekühlt wird und dabei vorzugsweise nicht umgelenkt wird.

4. Verfahren nach einem der vorstehenden Ansprüche, **dadurch gekennzeichnet, dass** die Pumpe und die Wärmetauscherflächen so ausgelegt werden, dass starke Temperaturschwankungen im Reaktor und ein zu starker Energieeintrag durch die Pumpe vermieden wird.

5. Verfahren nach einem der vorstehenden Ansprüche, **dadurch gekennzeichnet, dass** die Pumpe eine Seitenkanalpumpe ist.

6. Verfahren nach einem der vorstehenden Ansprüche, **dadurch gekennzeichnet, dass** die Zugabe des Acrylnitrils in dem Umpumpkreislauf kontinuierlich erfolgt.

7. Verfahren nach Anspruch 6, **dadurch gekennzeichnet, dass** die Zugabe des Acrylnitrils mit einer vorzugsweise frequenzgesteuerten Kolben-Membranpumpe erfolgt.

8. Verfahren nach einem der vorstehenden Ansprüche, **dadurch gekennzeichnet, dass** der Reaktorinhalt, bestehend aus Wasser und Biokatalysator, vor dem Reaktionsstart auf die Reaktionstemperatur gebracht wird.

9. Verfahren nach einem der vorstehenden Ansprüche, **dadurch gekennzeichnet, dass** nach Beendigung der Acrylnitrildosierung die Kühlung vorzugsweise mit einem Bypass reduziert wird.

10. Verfahren nach Anspruch 9, **dadurch gekennzeichnet, dass** die Reaktionszeit nach Beendigung der Acrylnitrildosierung noch 4 bis 20 Minuten, vorzugsweise 5 bis 10 Minuten, beträgt.

11. Verfahren nach einem der vorstehenden Ansprüche, **dadurch gekennzeichnet, dass** die On-Line-Messung mit einem FT-IR-Gerät erfolgt.

12. Verfahren nach einem der vorstehenden Ansprüche, **dadurch gekennzeichnet, dass** die Steuerung der Reaktion mit der On-Line-Messung erfolgt.

13. Verfahren nach Anspruch 12, **dadurch gekennzeichnet, dass** die Acrylnitrilzugabemenge, der Umpumpstrom, der Bypass und/oder die Nachreaktionszeit gesteuert werden.

14. Verfahren nach einem der vorstehenden Ansprüche, **dadurch gekennzeichnet, dass** der Biokatalysator Rhodococcus rhodochrous ist, der unter der Hinterlegungsbezeichnung 14230 bei der DSMZ, Deutsche Sammlung von Mikroorganismen und Zellkulturen GmbH, Mascheroder Weg 1 b, D-38124 Braunschweig, Deutschland, hinterlegt ist.

15. Vorrichtung zur Herstellung einer wässrigen Acrylamidlösung durch Hydratisierung von Acrylnitril in einer wässrigen Lösung in Gegenwart eines Biokatalysators mit einem Reaktor, einem Umpumpkreislauf, in dem ein Teil des Reaktorgemisches von einer Pumpe im Kreis gefördert wird und mindestens einem Wärmetauscher, der in dem Umpumpkreislauf angeordnet ist, wobei in dem Umpumpkreislauf unmittelbar vor dem Wiedereintritt des Reaktionsgemisches in den Reaktor eine Einspeisestelle für das Acrylnitril vorgesehen ist und wobei in dem Umpumpstrom ein On-Line-Messgerät zur Bestimmung der Acrylnitril- und/oder der Acrylamidkonzentration angeordnet ist.

16. Vorrichtung nach Anspruch 15, **dadurch gekennzeichnet, dass** der Reaktor eine Kühlung, vorzugsweise außenliegende Halbrohrschlangen, aufweist.

17. Vorrichtung nach Anspruch 15 oder 16, **dadurch gekennzeichnet, dass** der Wärmetauscher ein Rohrbündelwärmetauscher ist.

18. Vorrichtung nach einem der Ansprüche 15 bis 17, **dadurch gekennzeichnet, dass** die Pumpe eine Seitenkanalpumpe ist.

19. Vorrichtung nach einem der Ansprüche 15 bis 18, **dadurch gekennzeichnet, dass** der Wärmetauscher durch einen Bypass zumindest teilweise umgangen werden kann.

20. Vorrichtung nach einem der Ansprüche 15 bis 19, **dadurch gekennzeichnet, dass** das On-Line-Messgerät ein FT-IR-Gerät ist.

## Claims

1. Method for producing an aqueous acrylamide solution by hydrating acrylonitrile in an aqueous solution in the presence of a biocatalyst, wherein the reaction mixture is intermixed, the hydration is conducted in a reactor, which has a recirculation circuit, in which a part of the reaction mixture is circulated with a pump and in which at least one heat exchanger is arranged, **characterised in that** the addition of the acrylonitrile occurs in the recirculation circuit directly before the reaction mixture re-enters the reactor, and wherein the reaction course is monitored by on-line measurement by measuring the acrylonitrile and/or acrylamide concentration in the recirculation circuit before the addition point of the acrylonitrile.

2. Method according to claim 1, **characterised in that** the reactor has a cooling means, preferably external half pipe coil jackets.

3. Method according to claim 1 or 2, **characterised in that** the heat exchanger is a tubular heat exchanger, in which the reaction mixture is cooled and preferably not deflected during this.

4. Method according to one of the preceding claims, **characterised in that** the pump and the heat exchanger surfaces are designed so that severe temperature fluctuations are prevented in the reactor and too significant an energy supply through the pump is prevented.

5. Method according to one of the preceding claims, **characterised in that** the pump is a side channel pump.

6. Method according to one of the preceding claims, **characterised in that** the addition of the acrylonitrile occurs continuously in the recirculation circuit.

7. Method according to claim 6, **characterised in that** the addition of the acrylonitrile occurs with a preferably frequency-controlled piston-membrane pump.

8. Method according to one of the preceding claims, **characterised in that** the reactor content consisting of water and biocatalyst is brought to the reaction temperature before the start of the reaction.

9. Method according to one of the preceding claims, **characterised in that** after the dosing of the acrylonitrile has finished, the cooling is preferably reduced with a bypass.

10. Method according to claim 9, **characterised in that** the reaction time after the dosing of the acrylonitrile has finished still amounts to 4 to 20 minutes, preferably 5 to 10 minutes.

11. Method according to one of the preceding claims, **characterised in that** the on-line measurement is conducted with an FTIR device.

12. Method according to one of the preceding claims, **characterised in that** the control of the reaction occurs with the on-line measurement.

13. Method according to claim 12, **characterised in that** the amount of acrylonitrile added, the recirculation current, the bypass and/or the subsequent reaction time are controlled.

14. Method according to one of the preceding claims, **characterised in that** the biocatalyst is Rhodococcus rhodochrous, which is deposited under deposit reference 14230 at the DSMZ, Deutsche Sammlung von Mikroorganismen und Zellkulturen GmbH [German Collection of Microorganisms and Cell Cultures], Mascheroder Weg 1b, D-38124 Braunschweig, Germany.

15. Device for producing an aqueous acrylamide solution by hydrating acrylonitrile in an aqueous solution in the presence of a biocatalyst, with a reactor, a recirculation circuit, in which a part of the reaction mixture is circulated by a pump and at least one heat exchanger, which is arranged in the recirculation circuit, wherein a feed point for the acrylonitrile is provided in the recirculation circuit directly before the re-entry of the reaction mixture into the reactor, and wherein an on-line measuring device is arranged in the recirculation current to determine the acrylonitrile and/or the acrylamide concentration.

16. Device according to claim 15, **characterised in that** the reactor has a cooling means, preferably external half pipe coil jackets.

17. Device according to claim 15 or 16, **characterised in that** the heat exchanger is a tubular heat exchanger.

18. Device according to one of claims 15 to 17, **characterised in that** the pump is a side channel pump.

19. Device according to one of claims 15 to 18, **characterised in that** the heat exchanger can be bypassed at least partially by a bypass.

20. Device according to one of claims 15 to 19, **characterised in that** the on-line measuring device is an FTIR device.

## Revendications

1. Procédé de fabrication d'une solution aqueuse d'acrylamide par hydrogénation d'acrylonitrile en solution aqueuse en présence d'un biocatalyseur, dans lequel le mélange de réaction est brassé, l'hydrogénation est réalisée dans un réacteur qui présente un circuit fermé de recirculation dans lequel une partie du mélange de réaction est transportée en circuit fermé à l'aide d'une pompe et dans lequel au moins un échangeur de chaleur est disposé,
**caractérisé en ce que**
l'addition de l'acrylonitrile dans le circuit fermé de recirculation s'effectue immédiatement avant le retour du mélange de réaction dans le réacteur, l'évolution de la réaction étant surveillée par mesure en ligne en mesurant la concentration en acrylonitrile et/ou en acrylamide dans le circuit fermé de recirculation en amont de l'emplacement d'addition de l'acrylonitrile.

2. Procédé selon la revendication 1, **caractérisé en ce que** le réacteur présente un refroidissement, de préférence un serpentin semi-tubulaire extérieur.

3. Procédé selon les revendications 1 ou 2, **caractérisé en ce que** l'échangeur de chaleur est un échangeur de chaleur à faisceau de tubes dans lequel le mélange de réaction est refroidi de préférence sans y être recirculé.

4. Procédé selon l'une des revendications précédentes, **caractérisé en ce que** la pompe et les surfaces de l'échangeur de chaleur sont conçues de manière à éviter de fortes variations de températures dans le réacteur et un apport d'énergie trop intense par la pompe.

5. Procédé selon l'une des revendications précédentes, **caractérisé en ce que** la pompe est une pompe à canal latéral.

6. Procédé selon l'une des revendications précédentes, **caractérisé en ce que** l'addition de l'acrylonitrile dans le circuit fermé de recirculation s'effectue en continu.

7. Procédé selon la revendication 6, **caractérisé en ce que** l'addition de l'acrylonitrile s'effectue à l'aide d'une pompe à piston et membrane, de préférence commandée en fréquence.

8. Procédé selon l'une des revendications précédentes, **caractérisé en ce que** le contenu du réacteur, constitué d'eau et du biocatalyseur, est amené à la température de réaction avant le début de la réaction.

9. Procédé selon l'une des revendications précédentes, **caractérisé en ce que** lorsque l'addition d'acrylonitrile est terminée, le refroidissement est réduit de préférence en recourant à un contournement.

10. Procédé selon la revendication 9, **caractérisé en ce que** la durée de réaction après la fin de l'addition d'acrylonitrile est encore de 4 à 20 minutes et de préférence de 5 à 10 minutes.

11. Procédé selon l'une des revendications précédentes, **caractérisé en ce que** la mesure en ligne s'effectue à l'aide d'un appareil FT-IR.

12. Procédé selon l'une des revendications précédentes, **caractérisé en ce que** le contrôle de la réaction s'effectue à l'aide de la mesure en ligne.

13. Procédé selon la revendication 12, **caractérisé en ce que** la quantité ajoutée d'acrylonitrile, le débit de recirculation, le contournement et/ou la durée de fin de réaction sont contrôlés.

14. Procédé selon l'une des revendications précédentes, **caractérisé en ce que** le biocatalyseur est le rhodococcus rhodochrous déposé sous la désignation de dépôt 14 230 auprès de la DSMZ, Deutsche Sammlung von Mikroorganismen und Zellkulturen GmbH, Mascheroder Weg 1b, D-38124 Braunschweig, Allemagne.

15. Dispositif de fabrication d'une solution aqueuse d'acrylamide par hydrogénation d'acrylonitrile dans une solution aqueuse en présence d'un biocatalyseur, doté d'un réacteur, d'un circuit fermé de recirculation dans lequel une partie du mélange de réaction est transportée en circuit fermé au moyen d'une pompe et d'au moins un échangeur de chaleur disposé dans le circuit fermé de recirculation, un emplacement d'injection d'acrylonitrile étant prévu dans le circuit fermé de recirculation immédiatement en amont du retour du mélange de réaction dans le réacteur, un appareil de mesure en ligne destiné à déterminer la concentration en acrylonitrile et/ou en acrylamide étant disposé dans l'écoulement de recirculation.

16. Dispositif selon la revendication 15, **caractérisé en ce que** le réacteur présente un refroidissement, de préférence un serpentin semi-tubulaire extérieur.

17. Dispositif selon les revendications 15 ou 16, **caractérisé en ce que** l'échangeur de chaleur est un échangeur de chaleur à faisceau de tubes.

18. Dispositif selon l'une des revendications 15 à 17, **caractérisé en ce que** la pompe est une pompe à canal latéral.

19. Dispositif selon l'une des revendications 15 à 18, **caractérisé en ce que** l'échangeur de chaleur peut être contourné au moins partiellement par un contournement.

20. Dispositif selon l'une des revendications 15 à 19, **caractérisé en ce que** l'appareil de mesure en ligne est un appareil FT-IR.
